Europäisches Patentamt

European Patent Office

Office européen des brevets

(19)

(11) Veröffentlichungsnummer: **0 149 001**
**B1**

(12) # EUROPÄISCHE PATENTSCHRIFT

(45) Veröffentlichungstag der Patentschrift:
**24.06.87**

(21) Anmeldenummer: **84110952.3**

(22) Anmeldetag: **13.09.84**

(51) Int. Cl.⁴: **A 61 M 1/28**

(54) **Peritonealdialysegerät.**

(30) Priorität: **15.09.83 DE 3333362**

(43) Veröffentlichungstag der Anmeldung:
**24.07.85 Patentblatt 85/30**

(45) Bekanntmachung des Hinweises auf die Patenterteilung:
**24.06.87 Patentblatt 87/26**

(84) Benannte Vertragsstaaten:
**DE FR GB IT SE**

(56) Entgegenhaltungen:
**DE - A - 2 838 414**
**DE - A - 3 131 075**
**US - A - 4 190 047**
**US - A - 4 338 190**

(73) Patentinhaber: **Fresenius AG, Gluckensteinweg 5,
D-6380 Bad Homburg (DE)**

(72) Erfinder: **Polaschegg, Hans-Dietrich, Dr.,
Grünwiesenweg 9, D-6370 Oberursel 4 (DE)**

(74) Vertreter: **Luderschmidt, Wolfgang, Dr. Dipl-Chem.,
Görtz, Dr. Fuchs, Dr. Luderschmidt Patentanwälte
Sonnenberger Strasse 100 Postfach 26 26,
D-6200 Wiesbaden (DE)**

**Beschreibung**

Die Erfindung betrifft ein Peritonealdialysegerät gemäss dem ersten Teil des Anspruchs 1.

Bei einem Peritonealdialysegerät muss die zugeführte Peritonealspülflüssigkeit absolut steril sein, da es ansonsten zu der gefürchteten Peritonitis kommt. Derartige Sterilitätsprobleme treten üblicherweise bei der Hämodialyse nicht auf, da das Blut über ein eigenes Abwehrsystem verfügt, das eindringende Keime normalerweise beseitigt. Ein solches Abwehrsystem ist jedoch nicht im Peritoneum vorhanden, mit der Folge, dass die Peritonealdialyse unter den vorstehend erwähnten absolut sterilen Bedingungen durchgeführt werden muss.

Eine Vorrichtung der eingangs erwähnten Art ist aus der US-A-4 190 047 bekannt. Bei diesem Peritonealdialysegerät ist der die Dialysierflüssigkeit bereitstellende Kreislauf mit dem mit dem Peritoneum verbundenen Kreislauf durch einen Dialysator verbunden, dessen Membran somit eine Bakteriensperre darstellt.

Das bekannte Gerät soll die im Peritonealraum befindliche Spülflüssigkeit kontinuierlich von den Stoffwechselprodukten befreien, d.h. es soll nicht die gesamte Spülflüssigkeit, wie bei der CAPD üblich, nach einem bestimmten Intervall durch eine frische Spülflüssigkeit ersetzt werden. Bei dieser sekundären Dialyse wird jedoch nicht vollständig sterile Dialysierflüssigkeit an dem Dialysator entlanggeführt, der während des Betriebs nicht auf seine Dichtigkeit überprüft werden kann. Infolgedessen wird ein Patient zwangsläufig mit Keimen infiziert, wenn ein Riss, also ein erster Fehler, in der Membran des Dialysators auftritt, was durchaus nicht unüblich ist. Dies hat zur Folge, dass dieses Gerät aufgrund seiner nicht ausreichenden Sicherheit gegen einen ersten beliebigen Fehler aus Sicherheitsgründen nicht eingesetzt werden kann.

Aus «DIALYSIS + TRANSPLANTATION», Vol. 12, 1983, S. 385, ist ein Peritonealdialysegerät vorgeschlagen worden, das über einen Peritonealdialyseflüssigkeitskreislauf und über einen sekundären, nicht sterilen Dialysierflüssigkeitskreislauf verfügt, wobei beide Flüssigkeitskreisläufe durch ein Hämodialysefilter voneinander getrennt sind. An der Membran des Hämodialysefilters erfolgt die Abscheidung der in der Peritonealdialysierflüssigkeit enthaltenen Stoffwechselprodukte durch Diffusion durch die Membran in die nicht-sterile Dialysierflüssigkeit. Demzufolge kommt es zu einer dauernden Entfernung von harnpflichtigen Substanzen, nicht jedoch von osmotisch durch das Peritoneum freigesetztem Wasser.

Die in dieser Veröffentlichung beschriebene Peritonealdialyseapparatur weist folgende Nachteile auf:

Der Peritonealdialysekreislauf ist mit einem doppellumigen Katheter verbunden, so dass zugleich ein Einpumpen und Abpumpen von Flüssigkeit erfolgen kann und somit zu befürchten ist, dass eine bestimmte Flüssigkeitsmenge sofort nach dem Einpumpen wieder abgepumpt wird und dadurch dauernd der Dialyse entzogen ist. Hierdurch wird bei dieser stetig betriebenen Maschine die Dialysierdauer verlängert, also das Peritoneum häufig länger als erwünscht belastet.

Weiterhin wird gemäss dieser Veröffentlichung ein doppellumiger Katheter eingesetzt, der nur bei dieser Vorrichtung, nicht jedoch für andere Behandlungsmethoden einsetzbar ist. So kann die darin beschriebene Peritonealdialyseapparatur nicht bei einem Patienten eingesetzt werden, der der kontinuierlichen ambulanten Peritonealdialyse (CAPD) unterzogen wird. Da bei dieser Methode ein einlumiger Katheter eingesetzt wird, scheidet ein Dauerbetrieb des Peritonealdialysekreislauf aus. Infolgedessen können CAPD-Patienten nicht an eine derartige Vorrichtung angeschlossen werden.

Des weiteren ist bei der bekannten Vorrichtung keine Ultrafiltration bei sekundärer Dialyse möglich und darüber hinaus zu befürchten, dass bei der üblicherweise eingesetzten Single-Pass-Vorrichtung dies u.U. zu einer Infusion in den Peritonealdialysekreislauf kommen kann, so dass der Peritonealraum mit einer zusätzlichen Flüssigkeitsmenge gefüllt wird, was höchst unerwünscht ist.

Schliesslich ist die bekannte Maschine nicht ausreichend gegen einen ersten Fehler sicher. Die bekannte Vorrichtung soll die im Peritonealraum befindliche Spülflüssigkeit stets von den Stoffwechselprodukten befreien, d.h. es soll nicht die gesamte Spülflüssigkeit, wie bei der CAPD üblich, nach einem bestimmten Intervall durch eine frische Spülflüssigkeit ersetzt werden. Bei dieser sekundären Dialyse wird jedoch nicht-sterile Dialysierflüssigkeit an einem Hämodialysefilter entlanggeführt, das während des Betriebs nicht auf seine Dichtiggkeit überprüft werden kann. Infolgedessen wird ein Patient zwangsläufig mit Keimen infiziert, wenn ein Riss, also ein erster Fehler, im Hämodialysator auftritt, was durchaus nicht unüblich ist. Dies hat zur Folge, dass eine derartige Vorrichtung aus Sicherheitsgründen nicht eingesetzt werden kann.

Der Erfindung liegt daher die Aufgabe zugrunde, das Peritonealdialysegerät der eingangs erwähnten Art so fortzubilden, dass es als gegen einen beliebigen ersten Fehler sicher angesehen werden kann.

Die Lösung der Aufgabe erfolgt durch das kennzeichnende Merkmal des Anspruchs 1.

Das erfindungsgemässe Peritonealdialysegerät ist gegen einen ersten auftretenden Fehler, insbesondere gegen Membranbruch als sicher anzusehen, da es sehr unwahrscheinlich ist, dass zugleich zwei Fehler, also zwei Membranbrüche in unterschiedlichen Dialysefiltern vorkommen können.

Die Anordnung eines derartigen zweiten Filters hat darüber hinaus noch den Vorteil, dass ein in sich geschlossener Kreislauf entsteht, der im intakten Zustand gegenüber Keimen abgeschlossen ist und somit auch als eigensteril anzusehen ist.

Infolgedessen muss lediglich Sorge getragen werden, dass die vom zweiten Filter abgehende Leitung steril bleibt, damit der Patient immer wieder an diese Anordnung angeschlossen werden kann.

Das erfindungsgemässe Peritonealdialysegerät kann von einem Patienten im Gegensatz zu der üblichen Hämodialyse ohne Aufsicht betrieben werden, da es infolge seiner Betriebssicherheit keine Aufsichtsprobleme aufwirft.

Des weiteren werden sicher im Peritoneum freigesetzte Proteine zurückgehalten und bleiben im Peritoneum, wo sie wiederum resorbiert werden.

Gegenüber der CAPD-Behandlung ist der Betrieb der erfindungsgemässen Peritonealdialysemaschine erheblich billiger, so dass mehr Dialysierflüssigkeit eingesetzt werden kann, mit der Folge, dass die Behandlungszeit erheblich verkürzt wird. Infolgedessen reicht üblicherweise eine Behandlung des Patienten über Nacht aus, so dass der Patient am Tag sich frei bewegen kann.

Schliesslich kann auch die unbeständige Bicarbonat-Dialysierflüssigkeit unmittelbar von der Peritonealdialysemaschine hergestellt und dem Patienten zur Verfügung gestellt werden. Infolgedessen können Lactat oder Acetat enthaltende Dialysierflüssigkeiten, die weniger verträglich sind, durch die Bicarbonat enthaltende Dialysierflüssigkeit ersetzt werden.

Weiterhin kann mit dem erfindungsgemässen Peritonealdialysegerät auf der Seite der Bereitstellung der Dialysierflüssigkeit mittels einer Einrichtung zur Entziehung von Ultrafiltrat eine entsprechende Menge Wasser dem Patienten entzogen werden, wobei dies wahlweise gesteuert oder nicht gesteuert erfolgen kann.

In nichtgesteuerter Weise wird dem Patienten anhand von empirisch ermittelten Werten die entsprechende Menge Ultrafiltrat während der Behandlung entzogen. Dabei fliesst die restliche Menge Ultrafiltrat nach dem Entkuppeln des Konnektors in einen Beutel ab.

Andererseits kann jedoch auch die Ultrafiltration gesteuert durchgeführt werden, wobei erfindungsgemäss zwei Varianten eingesetzt werden.

Die erste Variante sieht den Einsatz eines druckempfindlichen Gürtels vor, der um den Bauch des Patienten gelegt ist. Die durch die Osmose erzeugte Ultrafiltratmenge bewirkt dabei eine Vergrösserung des Bauchumfangs und hierdurch eine Spannung im Gürtel, die einen entsprechenden Detektor betätigt. Dieser druckempfindliche Detektor löst eine Steuerungseinrichtung aus, die die Ultrafiltratpumpe betätigt.

Eine andere Variante nutzt dagegen die Vergrösserung der Wassermenge in dem abgeschlossenen Raum, der aus dem Peritoneum einerseits und dem geschlossenen extrakorporalen Kreislauf andererseits besteht. In diesem geschlossenen Raum wird mit wachsender Ultrafiltratmenge die Konzentration einer Substanz gesenkt, die gelöst oder suspendiert in der Dialysierflüssigkeit vorgesehen ist und mittels eines Detektors feststellbar ist. Der sich bei der Peritonealdialyse einstellende Konzentrationsgradient kann daher zur Steuerung der Ultrafiltration eingesetzt werden, wobei lediglich die ursprüngliche Konzentration und die Konzentration bei der Behandlung miteinander verglichen werden müssen. In einer üblichen Komparatorschaltung kann dann die Ultrafiltratpumpe so lange betrieben werden, bis die ursprüngliche Konzentration der zu detektierenden Substanz wieder erreicht ist, d.h. die osmotisch erzeugte Ultrafiltratpumpe abgepumpt ist.

Vorteilhafterweise weist die Vorrichtung zur Bereitstellung der Dialysierflüssigkeit einen Konzentratbehälter auf, der die osmotisch wirkende Substanz, insbesondere Glucose, konzentriert enthält. Diese osmotisch wirkende Substanz wird der die üblichen Elektrolytmengen enthaltenden Dialysierflüssigkeit in den gewünschten osmotisch wirksamen Mengen zugesetzt und zum Hämodialysator gepumpt. Hierin erfolgt über die Membran hinweg der Ausgleich der osmotisch wirksamen Menge, die teilweise im Peritoneum resorbiert wird und daher nachgeführt werden muss. Im Gegensatz hierzu soll jedoch die zur Steuerung der Ultrafiltration eingesetzte Substanz im wesentlichen nicht im Peritoneum resorbiert oder verändert werden.

Weitere Einzelheiten, Merkmale und Vorteile sind aus der nachfolgenden Beschreibung von Ausführungsbeispielen unter Bezugnahme auf die Zeichnung ersichtlich.

Es zeigen:

Fig. 1 ein Schema einer ersten Ausführungsform des erfindungsgemässen Peritonealdialysegerätes,

Fig. 2 ein Schema einer zweiten Ausführungsform des erfindungsgemässen Peritonealdialysegerätes,

Fig. 3 eine schematische Darstellung einer dritten Ausführungsform, bei der lediglich eine Bilanzkammer zum Einsatz kommt, und

Fig. 4 ein Schema einer vierten Ausführungsform eines Peritonealdialysegerätes mit einem Steuergerät, mit dem spezielle Füll-, Desinfektions- und Prüfprogramme durchgeführt werden können.

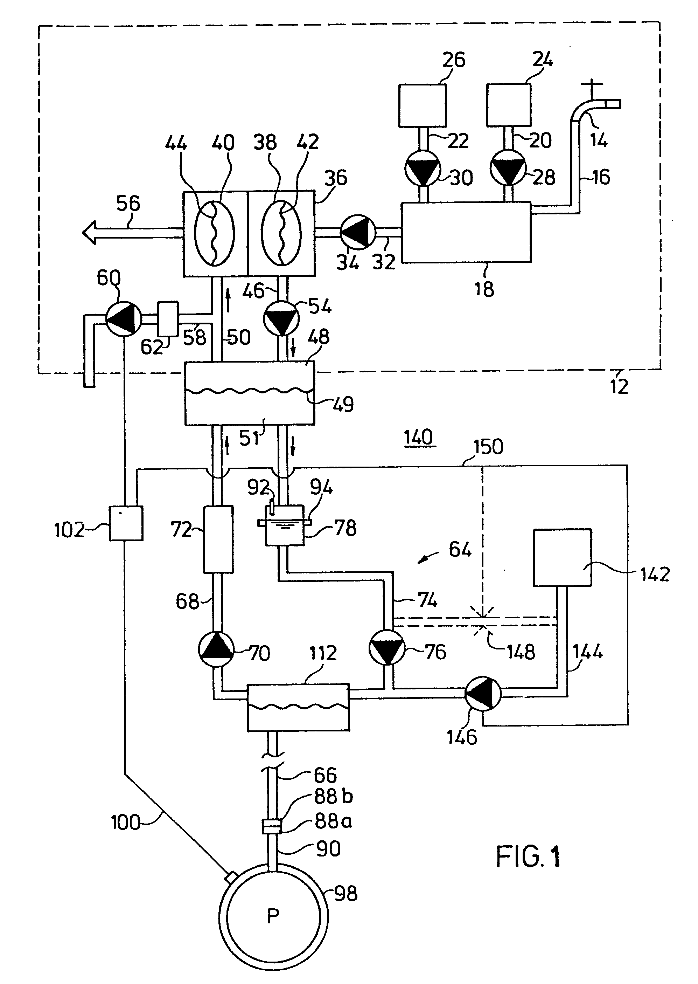

In Fig. 1 ist mit 140 das erfindungsgemässe Peritonealdialysegerät gezeigt, das vorteilhafterweise eine Einrichtung zur Bereitstellung der Dialysierflüssigkeit 12 aufweist, was in Fig. 1 gestrichelt dargestellt ist. Diese Einrichtung 12 ist vorteilhafterweise in einer üblichen Hämodialysevorrichtung vorgesehen, wie sie beispielsweise von der Anmelderin unter der Bezeichnung A 2008 vertrieben wird. Eine derartige Hämodialysevorrichtung wird vorteilhafterweise im sogenannten Single-Pass betrieben, d.h. die Dialysierflüssigkeit wird nur einmal durch den Dialysator geschickt und anschliessend verworfen. Weiterhin wird diese Einrichtung vorteilhafterweise volumengesteuert nach dem sogenannten Bilanzkammerprinzip betrieben, wie dies in der DE-A-2 838 414 beschrieben ist.

In Fig. 1 ist diese bilanzierende Vorrichtung schematisch dargestellt. Die Einrichtung 12 weist dementsprechend einen Frischwasseranschluss 14 auf, der über eine Leitung 16 mit einer Einheit 18 zur Erzeugung der Dialysierflüssigkeit verbunden ist.

Die Einheit 18 ist weiterhin über Leitungen 20, 22 mit dem das Elektrolytkonzentrat enthaltenden Behälter 24 bzw. dem das Konzentrat der osmotisch wirksamen Substanz enthaltenden Behälter 26 verbunden. In die Leitungen 20 und 22 sind jeweils Pumpen 28 und 30 eingeschaltet, die die Konzentrate im Umschalttakt chargenweise zufördern, der ebenfalls, wie nachstehend erläutert, die Bilanzkammern steuert. Die Pumpe 30 zur Förderung des Konzentrats der osmotisch wirksamen Substanz, die vorteilhafterweise Glucose ist, kann vorteilhafterweise entsprechend der in der Dialysierflüssigkeit gewünschten Konzentration der osmotisch wirksamen Substanz eingestellt werden. Üblicherweise liegt die Konzentration der Glucose dabei in der fertigen Dialysierflüssigkeit in einem Bereich von 1,5 - 4,25%, was einer theoretischen Osmolarität von etwa 358 bis 516 mosm/l entspricht. Gewöhnlich wird dabei das Konzentrat der osmotisch wirksamen Substanz in ähnlicher Weise wie das Elektrolytlösungskonzentrat um den Faktor 35 verdünnt, d.h. das Konzentrat muss um einen entsprechenden Faktor angereichert sein.

Die Einheit 18 weist weiterhin eine übliche nicht gezeigte Erwärmungs- und Entgasungseinrichtung auf, mit der die Dialysierflüssigkeit erwärmt und entgast wird.

Weiterhin ist die Einheit 18 über eine Leitung 32, in die eine Förderpumpe 34 eingeschaltet ist, mit der Bilanzkammergruppe 36 verbunden, die in Fig. 1 nur schematisch dargestellt ist. Vorteilhafterweise enthält die Bilanzkammergruppe zwei Bilanzkammern 38 und 40, die durch eine Membran 42 und 44 in die jeweiligen Bilanzkammerhälften geteilt sind. Die Art und Weise des Betriebs dieser Bilanzkammern ist ebenfalls in der DE-A-2 838 414 beschrieben.

Die Bilanzkammergruppe 36 ist über eine Leitung 46 mit dem Einlass eines Dialysators 48 verbunden, während der Auslass des Dialysators 48 über die Leitung 50 mit der Bilanzkammergruppe 36 verbunden ist. Somit entsteht ein geschlossener Kreislauf zwischen der einen Kammer des Dialysators 48 über die Leitungen 46 und 50 mit der Bilanzkammergruppe 36.

Im Betrieb werden die Bilanzkammern 38 und 40 jeweils entweder mit der Dialysierflüssigkeit gefüllt und zugleich von gebrauchter Dialysierflüssigkeit entleert oder die bereits mit Dialysierflüssigkeit gefüllte Kammer wird mittels einer in die Leitung 46 eingeschalteten Pumpe 54 entleert, wobei die gebrauchte Dialysierflüssigkeit in die andere Bilanzkammerhälfte zurückgeführt wird. Sobald sich die Membranen 42, 44 an die Wand der Bilanzkammern 38, 40 angelegt haben, erfolgt die Umschaltung über entsprechende, nicht gezeigte Ventile, so dass sich die Betriebsweise der Bilanzkammern umkehrt. Die Abförderung der verbrauchten Flüssigkeit erfolgt dabei über die Leitung 56.

Um diesem geschlossenen System Ultrafiltrat zu entnehmen, ist die Leitung 50 mit einer Leitung 58 verbunden, in die eine Ultrafiltratpumpe 60 eingeschaltet ist. Die hierbei entnommene Flüssigkeit wird vorteilhafterweise in einem Entgasungsgefäss 62 entgast, wie dies ebenfalls in der DE-A-2 838 414 beschrieben ist. Demgemäss arbeitet die UF-Pumpe 60 vorteilhafterweise volumetrisch, wobei insbesondere eine Kolben- oder Membranpumpe zum Einsatz kommen kann.

Als Dialysator 48 kommt vorteilhafterweise ein üblicher Hämodialysator in Frage, der als Platten- oder Hohlfaserdialysator ausgebildet sein kann. Derartige Dialysatoren besitzen eine Durchlassgrenze von ca. 5000 - 10 000 MG und verhindern somit im unversehrten Zustand den Durchtritt von Bakterien von der Seite der Membran, aus der die Zuführung der frisch hergestellten Dialysierflüssigkeit erfolgt, also von der Seite der Einrichtung 12. Demzufolge muss nicht unbedingt absolut sterile Dialysierflüssigkeit eingesetzt werden, da die Sterilfiltrierung an dem Hämodialysefilter erfolgen kann. Andererseits kann jedoch auch ein übliches Hämofilter eingesetzt werden, dessen Trenngrenze unterhalb des Molekulargewichts von Albumin (60 000 MG) liegt, wodurch das bei der CAPD-Behandlung unvorteilhafte Entfernen dieses Proteins vermieden wird.

Auf der anderen Seite der Membran des Dialysators 48 ist ein Kreislauf 64 vorgesehen, der nur von einer Leitung 66 her beschickt werden kann. Der Kreislauf 64 weist eine Leitung 68 auf, die an ihrem anderen Ende mit dem Einlass des Dialysators 48 verbunden ist. In diese Leitung 68 ist eine Pumpe 70, vorzugsweise eine peristaltische Pumpe, sowie ein Speichergefäss 72 eingeschaltet.

Vom Dialysator 48 geht auf dieser Seite der Membran eine weitere Leitung 74 ab, in die ebenfalls eine Pumpe 76 sowie gegebenenfalls eine Tropfkammer 78 eingeschaltet ist.

Die Pumpen 70 und 76 in den Leitungen 68 bzw. 74 sind gemäss der in Fig. 1 gezeigten Ausführungsform vorteilhafterweise als peristaltische Pumpen ausgebildet.

Die Ausführungsform weist ein Dialysefilter 112 auf, dessen Auslass mit dem einen Ende der Leitung 68 verbunden ist, die mit ihrem anderen Ende mit dem Dialysator 48 verbunden ist. Der Einlass des Dialysefilters 112 steht weiterhin mit dem Ende der Leitung 74 in Verbindung, die sich bis zum Auslass des Dialysators 48 erstreckt.

Die Leitung 66 ist mit dem Filtratauslass des Dialysefilters 112 verbunden, so dass also ein völlig geschlossener Kreislauf gebildet wird, der aus der Leitung 68, der ersten Membrankammer des Dialysators 48, der Leitung 74 und der ersten Kammer des Dialysefilters 112 besteht. Lediglich durch die Poren der Membranen des Dialysators 48 und des Dialysefilters 112 besteht die Möglichkeit des Stoffaustausches unter Ultrafiltration. Vorzugsweise sind die Porengrössen dieser Membranen derart beschaffen, dass ein Eindringen von Keimen in diesen geschlossenen Kreislauf nicht möglich ist. Wie bereits vorstehend erwähnt, weist der übliche Dialysator eine Porengrösse von etwa 30 - 80 Å auf, während die Dialysefilter 112 die gleiche Porengrössenverteilung oder aber erheblich darüberliegende Porengrössen aufweist. Bevorzugt ist dabei ein Dialysefilter 112, das eine erheblich grössere mittlere Porengrössenverteilung aufweist, beispielsweise Moleküle mit einem Molekulargewicht (MG) von wenigstens 30 000 nicht mehr durchlässt. Die obere Grenze derartiger Porengrössen ist die Keime sicher zurückhaltende Porengrösse, die etwa zwischen 0,2 und 0,5 µm liegt.

Ein Beispiel für ein derartiges Filter 112 ist der bei der Hämofiltration eingesetzte Hämofilter, der eine Trenngrenze von etwa 30 000 MG besitzt, also sicher noch Albumin mit 60 000 MG zurückhält.

Falls jedoch das Albumin nicht zurückgehalten werden soll, können auch Kaskadenfilter eingesetzt werden, die eine Trenngrenze von etwa 200 000 bis 400 000 MG aufweisen.

Besonders bevorzugt ist eine Anordnung gemäss Fig. 1, die einerseits einen Hämodialysator als Dialysator 48 mit einer Trenngrenze von 5000 - 10 000 MG besitzt und andererseits einen Hämofilter als Dialysefilter 112 aufweist, dessen Trenngrenze in einem Bereich von 20 000 - 400 000 MG liegt.

Als Speichergefäss 72 kommt vorteilhafterweise ein schlauchartiges Gefäss in Frage, dessen Wände kollabierbar sind. Ein solcher Schlauch kann beispielsweise in Form eines Beutels ausgebildet sein.

In einer ersten Ausführungsform ist die Wand dieses Speichergefässes 72 zwar kollabierbar, jedoch nicht erweiterbar. Ein derartiges Speichergefäss ist beispielsweise in der US-A-3 791 767 beschrieben.

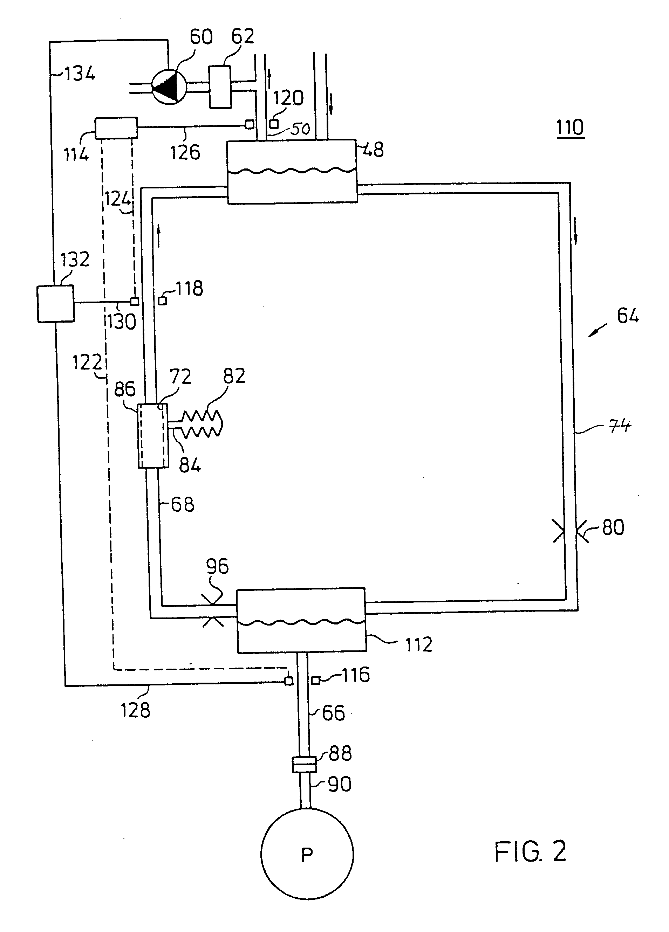

In einer zweiten Ausführungsform lässt sich die Wand des kollabierbaren Speichergefässes elastisch erweitern und geht nach entsprechender Entlastung wieder in seinen Ausgangszustand zurück. Demzufolge kann anstelle der Pumpe 76, die zur Entleerung des Speichergefässes 72 dient, auch eine in Fig. 2 gezeigte Klemme 80 eingesetzt werden, die im Gegentakt zur Pumpe 70 arbeitet. Ein derartiges Speichergefäss 72 mit erweiterbarer Wand ist beispielsweise in der DE-A-3 131 075 offenbart.

Das Speichergefäss 72 soll im Speicherzustand etwa 300 - 700, insbesondere etwa 500 ml Dialysierflüssigkeit aufnehmen. Demzufolge muss ein Speichergefäss mit nicht erweiterbarer Wand wenigstens ein solches Innenvolumen zur Verfügung stellen, während ein Speichergefäss mit elastischer Wand hinsichtlich seines Innenvolumens kleiner ausfallen kann, beispielsweise um den Faktor 2 - 3 in seinem Volumen geringer sein kann.

Anstelle der Pumpe 70 kann vorteilhafterweise eine Balgpumpe 82 eingesetzt werden, wie dies in Fig. 2 gezeigt ist. Die Balgpumpe 82 ist über eine Leitung 84 mit dem Gehäuse 86 verbunden, in dem das Speichergefäss 72 angeordnet ist. Dieses Speichergefäss 72 kann mit nicht erweiterbarer Wand ausgebildet sein, weist jedoch vorteilhafterweise eine erweiterbare Wand auf. Während die erste Anordnung in der US-A-3 791 767 beschrieben ist, ist die zweite Anordnung in der DE-A-3 205 449 beschrieben.

Wie aus Fig. 1 ersichtlich ist, kann die Leitung 66 über einen Konnektor 88 mit dem Katheter 90 verbunden sein, der im Peritoneum des Patienten implantiert ist. Andererseits kann jedoch auch die Leitung 66 unmittelbar Bestandteil des Katheters 90 sein, beispielsweise mit dem Katheter verschweisst sein, ohne dass ein Konnektor 88 vorgesehen ist. In einem solchen Fall ist jedoch die Leitung 66 über eine spezielle Schlauchschweissmaschine vom Katheter reversibel abtrennbar.

Ein Konnektorsystem, das als Konnektor 88 eingesetzt werden kann, ist beispielsweise in der DE-A-2 947 574 beschrieben.

Durch Öffnen des Konnektors 88 und entsprechendes Verschliessen der Konnektorteile kann also der Patient von dem Peritonealdialysegerät 10 getrennt und zur Behandlung wieder angeschlossen werden.

Diese in Fig. 1 dargestellte, besonders bevorzugte Ausführungsform eines Peritonealdialysegerätes 140 kann zusätzlich mit einem Gefäss 142 verbunden sein, das eine Zusatzflüssigkeit enthält. Eine derartige Zusatzflüssigkeit wird dann eingesetzt, wenn die Dialysebehandlung des Patienten verbessert werden soll. So können beispielsweise besonders kostspielige Arzneimittel, wie Insulin, Chelatbildner, die Filtrationseigenschaften des Peritoneums verbessernde Substanzen und dgl., in einem derartigen Gefäss 142 steril vorgelegt werden.

Das Gefäss 142 ist über eine Leitung 144 stromab der Pumpe 76 mit der Leitung 74 verbunden, wobei in die Leitung 144 eine Pumpe 146 eingeschaltet ist.

Andererseits kann jedoch auch die Leitung 144 stromauf der Pumpe 76 mit der Leitung 74 verbunden sein, wie dies in Fig. 1 gestrichelt dargestellt ist. In einem solchen Fall ist anstelle der Pumpe 146 eine Klemme 148 vorgesehen, die die Leitung 144 entsprechend abklemmt oder öffnet, wie dies nachstehend erläutert ist.

Das in Fig. 1 gezeigte Peritonealdialysegerät 140 arbeitet auf folgende Weise:

Zunächst wird das Peritoneum des Patienten P mit steriler Dialysierflüssigkeit gefüllt, wie dies beispielsweise bei der CAPD der Fall ist. Hierzu wird ein die entsprechende Menge Dialysierflüssigkeit enthaltender Beutel über den Konnektor 88 an den Katheter 90 angeschlossen und entleert. Anschliessend wird diskonnektiert und der Patient über den Konnektor 88 an der Peritonealdialysegerät 10 angeschlossen, das bereits vollständig mit der entsprechenden Dialysierflüssigkeit gefüllt ist.

Die Dialysierflüssigkeit wird zunächst in der Einheit 12 aus den entsprechenden Konzentraten und Leitungswasser erzeugt und durch die Pumpe 34 der Bilanzkammergruppe 36 erwärmt und entgast zugeführt. Diese Bilanzkammergruppe 36 schickt die bereitgestellte Dialysierflüssigkeit in volumetrisch gesteuerter Weise durch den Dialysator 48, ohne dass zunächst ein Unterdruck zur Erzeugung der Ultrafiltration angelegt wird. Infolge der inkompressiblen Eigenschaften der Dialysierflüssigkeit kommt es daher zu keinem Abzug von Wasser aus dem gesamten geschlossenen System.

Auf der anderen Seite der Membran 49 wird die im Peritoneum und dem Kreislauf befindliche Dialysierflüssigkeit intermittierend umgepumpt. Hierzu wird zunächst die Pumpe 70 in Betrieb gesetzt, während die Pumpe 76 steht oder die Klemme 80 die Leitung 74 verschliesst. Die Pumpe 70 zieht also eine entsprechende Menge Flüssigkeit, beispielsweise 300 bis 700, insbesondere ca. 500 ml, ab und pumpt diese Flüssigkeitsmenge in das Speichergefäss 72. Anschliessend wird die Pumpe 70 abgeschaltet und sperrt somit den Rückfluss in der Leitung 68. Mit dem Abschalten der Pumpe 70 wird die Pumpe 76 in Betrieb gesetzt oder die Klemme 80 geöffnet. Hierauf wird die im Speichergefäss 72 befindliche Flüssigkeit abgepumpt und dabei an der Membran 49 des Dialysators 48 entlanggeführt. Da das Peritoneum als primäre Membran dient und durch Diffusion die entsprechenden Stoffwechselprodukte in die im Peritoneum befindliche Flüssigkeit aus dem Blut abgegeben hat, werden diese Stoffwechselprodukte ebenfalls an der Dialysemembran 49 vorbeigeführt und diffundieren infolge des Konzentrationsgradienten durch die Membran 49 und werden anschliessend mit der gebrauchten Dialysierflüssigkeit über die Leitung 56 in den Abfluss befördert. Nach der Diffusionsbehandlung gelangt die Dialysierflüssigkeit in eine Tropfkammer 78, die vorteilhafterweise ein hydrophobes Filter 92 aufweist, an dem die Luft abgeschieden werden kann und die die Flüssigkeitshöhe in der Tropfkammer 78 festlegt. Eine derartige Tropfkammer ist beispielsweise in der DE-A-3 115 299 beschrieben. An dem Aussenumfang der Tropfkammer ist ein Sensor 94 angeordnet, mit dem das Flüssigkeitsniveau in der Tropfkammer 78 festgestellt werden kann. Sinkt dieses Flüssigkeitsniveau unter eine bestimmte Grenze, so wird die Pumpe 76 abgestellt bzw. die Klemme 80 geschlossen.

Andererseits muss jedoch die Tropfkammer 78 nicht zwangsläufig vorgesehen sein. In diesem Fall wird die Pumpe 76 so lange betrieben, bis der Speicher 72 vollständig entleert ist. Nachdem die Dialysierflüssigkeit wieder in das Peritoneum des Patienten zurückgepumpt ist, beginnt von neuem die Entnahme von Dialysierflüssigkeit durch Einschalten der Pumpe 70 bzw. 82. Ist eine Pumpe 82 am Aussenumfang des Speichergefässes 72 vorgesehen, so ist in der Nähe des y-förmigen Übergangs eine Klemme 96 vorgesehen, wie dies in Fig. 2 gezeigt ist. Diese Klemme 96 weist dabei die Wirkung der stehenden Pumpe 70 auf, sperrt also die Leitung 68.

Die vorstehende Verfahrensweise betrifft lediglich die Reinigung des Patienten von Stoffwechselprodukten, nicht jedoch seine Entwässerung, die nur durch Ultrafiltration bewirkt werden kann.

Da an den Patienten selbst kein Unterdruck angelegt werden kann, erfolgt die Entnahme des Wassers primär durch die Osmose, wobei die in der Dialyseflüssigkeit enthaltene osmotisch wirksame Substanz, beispielsweise Glucose, in der vorstehend angegebenen Konzentration diese Ultrafiltration bewirkt. Das sich im Peritoneum sammelnde Wasser bewirkt eine Vergrösserung des Bauchumfangs, so dass ein auf diese Vergrösserung ansprechender Gürtel 98 zur Regulierung der Ultrafiltration eingesetzt werden kann. Dieser auf den Umfang ansprechende Gürtel 98 ist über eine Leitung 100 mit einem Ultrafiltrationsregelgerät 102 verbunden, das seinerseits die Ultrafiltratpumpe 60 steuert. Die Ultrafiltratpumpe 60 entnimmt daher in Abhängigkeit von dem Signal des Gürtels 98 eine bestimmte Menge Dialysierflüssigkeit und erzeugt durch seine Entnahme einen Unterdruck an der Membran 49, mit der Folge, dass die gleiche Menge Dialysierflüssigkeit aus dem Kreislauf 64 und in Verbindung damit aus dem Peritoneum entnommen wird.

Hinzuzufügen ist, dass die osmotisch wirksame Substanz, wie Glucose, Fructose, Mannose, Maltose und dgl., teilweise im Peritoneum resorbiert wird, so dass deren Konzentration bei der CAPD kontinuierlich absinkt. Mit dem erfindungsgemässen Peritonealdialysegerät wird jedoch das Absinken der Konzentration verhindert, da die Einrichtung 12 stets frische Dialysierflüssigkeit an der Membran 49 vorbeiführt, was bei einem an der Membran 49 vorliegenden Konzentrationsgradienten zu einem Konzentrationsausgleich auf der Seite des Peritoneums führt. Demgemäss bleibt die osmotisch wirksame Konzentration der osmotisch wirksamen Substanz während der Behandlung im wesentlichen aufrechterhalten.

Aus dem Gefäss 142 wird nun auf folgende Weise die Zusatzflüssigkeit entnommen:

Wenn die Pumpe 70 in Betrieb ist, also die Pumpe 76 steht bzw. bei der in Fig. 1 gezeigten Ausführungsform die Klemme 96 geöffnet und die Klemme 80 geschlossen ist, ist die Pumpe 146 ausser Betrieb bzw. die Klemme 148 geschlossen.

Beim Inbetriebsetzen der Pumpe 76 bzw. Öffnen der Klemme 80 und Abschalten der Pumpe 70 bzw. Schliessen der Klemme 96 wird die Pumpe 146 entsprechend einer vorgewählten Fördermenge in Betrieb gesetzt. Andererseits wird die Klemme 148 derart geöffnet, dass die gewünschte Menge Flüssigkeit aus dem Gefäss 142 entnommen werden kann. Bevorzugt ist jedoch der Einsatz einer Pumpe 146, da hierdurch eine genaue Zudosierung der zusätzlichen Flüssigkeit möglich ist.

Wie in Fig. 1 gezeigt, ist die Pumpe 146 bzw. die Klemme 148 über eine elektrische Leitung 150 mit dem Ultrafiltrationsregelgerät 102 verbunden. Gemäss dieser Ausführungsform wird nämlich durch die zusätzliche Zuführung einer Flüssigkeit aus dem Gefäss 142 die dem Patienten zugeführte Flüssigkeitsmenge kontinuierlich erhöht, was zu einer nachteiligen Überwässerung des Patienten führen kann.

Das Ultrafiltrationssteuergerät 102 betätigt zur Vermeidung dieser Probleme seinerseits die Ultrafiltrationspumpe 60 derart, dass sie eine derartige Menge an Flüssigkeit abzieht, die der von der Pumpe 146 zugeführten zusätzlichen Flüssigkeitsmenge entspricht. Dementsprechend wird bereits durch die Vorwahl der durch die Pumpe 146 zuzuführenden Flüssigkeitsmengen die Förderrate der Ultrafiltrationspumpe programmiert, mit der Folge, dass die Flüssigkeitsbilanz bei Zuführung einer zusätzlichen Flüssigkeit aus einem Gefäss 142 im wesentlichen ausgeglichen ist. Anzumerken ist, dass die Ultrafiltrationspumpe 60 diese Flüssigkeitsmenge natürlich zusätzlich zu der zu ultrafiltrierenden Menge abzieht, so dass im Beispielsfall die Ultrafiltrationspumpe doppelt programmiert werden muss, nämlich in der tatsächlich zu ultrafiltrierenden Menge und in der Menge, die dem Patienten aus dem Gefäss 142 zusätzlich zugeführt wird und die demzufolge zusätzlich abgeführt werden muss.

Andererseits kann die Ultrafiltrationspumpe 60 natürlich auch geregelt mit Hilfe des Gürtels 98 Ultrafiltrat entziehen, so dass eine derartige Vorprogrammierung unnötig ist.

Vorteilhafterweise wird man jedoch die Ultrafiltratpumpe 60 programmiert gesteuert einsetzen, wie dies nachstehend erläutert ist.

Die vom Peritoneum eines Patienten durch Osmose entwickelte Wassermenge hängt im wesentlichen von der Osmolarität, d.h. dem Gehalt der osmotisch wirkenden Substanz in der Dialysierflüssigkeit ab und ist darüber hinaus patientenspezifisch. Dementsprechend kann ein Patient nach entsprechender Bestimmung der von ihm entwickelten Ultrafiltratmenge bei Verwendung einer Dialysierflüssigkeit mit bestimmter Zusammensetzung von der Peritonealdialyse die Zusammensetzung der Dialysierflüssigkeit durch entsprechende Wahl der Menge der osmotisch wirksamen Substanz und somit indirekt die zu entwickelnde Ultrafiltratmenge bestimmen. Zu Beginn der Peritonealdialyse programmiert er also die Echtheit 18 zur Erzeugung der Dialysierflüssigkeit, die dann eine entsprechend zusammengesetzte Dialysierflüssigkeit zur Verfügung stellt. Des weiteren wird hierdurch zugleich die Ultrafiltrationspumpe 60 derart programmiert, dass im wesentlichen die vom Patienten entwickelte Ultrafiltratmenge abgezogen wird. Sofern die Ultrafiltrationspumpe 60 eine geringe Menge Flüssigkeit mehr oder weniger aus dem Peritonealraum abzieht, so schadet dies nicht, da dann entsprechend weniger Flüssigkeit beim Öffnen des Konnektors 88 aus dem Peritonealraum des Patienten abfliesst.

In Fig. 2 ist eine weitere bevorzugte Ausführungsform eines Peritonealdialysegerätes 110 gezeigt, wobei für gleiche Teile die gleichen Bezugszeichen, wie in Fig. 1 verwendet werden. Weiterhin ist aus Gründen der Übersichtlichkeit die Einrichtung 12 zur Bereitstellung von Dialysierflüssigkeit weggelassen.

Wie bereits vorstehend erläutert, handelt es sich bei dem System gemäss Fig. 2 um ein abgeschlossenes System, das auch im unbenutzten Zustand, d.h. also im entkoppelten Zustand, eigensteril ist. Demzufolge müssen also die kostspieligen Filter 48 und 112 nicht zwangsläufig nach jeder Behandlung ausgetauscht werden.

Um ein Brechen der Membranen in den Filtern 48 und 112 zu erkennen, ist in einer weiteren, in Fig. 2 gezeigten Ausführungsform eine Überwachungseinrichtung 114 vorgesehen, die mit den Sensoren 116, 118 und/oder 120 zusammenwirkt. Diese Sensoren sind über entsprechende Leitungen 122, 124 und 126 mit der Überwachungseinrichtung 114 verbunden.

Um ein Leck in den Membranen der Filter 48 und 112 zu erkennen, ist in dem geschlossenen Kreis, der zwischen den Filtern gebildet wird, eine von diesen Sensoren 116 - 120 erkennbare Substanz vorgesehen, deren Molekülgrösse so gross ist, dass das Molekül eine unbeschädigte Membran nicht durchdringen kann.

Bei einem üblichen Hämodialysator kommt hierfür Inulin, Myoglobin, Stärke, Hydroxyethylstärke, Polysaccharide, wie Dextrane, in Frage, die gegebenenfalls einen detektierbaren Molekülbestandteil aufweisen. Zu derartigen detektierbaren Molekülbestandteilen gehören beispielsweise fluoreszierende oder im sichtbaren Bereich absorbierende Molekülbestandteile, die an das Grundmolekül kovalent gebunden sind, beispielsweise Dextranblau. Solche Molekülbestandteile werden auch als Label gekennzeichnet.

Wie aus der Fig. 2 ersichtlich ist, sprechen die Detektoren 116 und 120 erst bei einem Durchtreten derartiger «Sicherheitssubstanzen» an, was zu dem Abschalten der gesamten Anordnung führt. Andererseits kann der Detektor 118 auf eine Verarmung, d.h. Konzentrationsabnahme derartiger Substanzen ansprechen, was ebenfalls zum Abschalten der Vorrichtung führt.

Wie bereits erwähnt, müssen also die Sicherungssubstanzen in ihrer Grösse so ausgelegt sein, dass sie sicher von den Membranen der Filter 48 und 112 zurückgehalten werden.

In einer weiteren Ausführungsform werden die Substanzen so gewählt, dass sie die Membran des Dialysators 48 nicht durchdringen können, jedoch durch die Membran des Dialysefilters 112 ohne Schwierigkeiten diffundieren können. In einem solchen Fall ist also das Molekulargewicht dieser Substanzen geringer als die Trenngrenze des Dialysefilters 112. Demzufolge kommt es zu einer Diffusion dieser Substanz durch die Membran des Dialysefilters 112 in das Peritoneum, so dass diese Substanz grundsätzlich nicht toxisch sein darf. Weiterhin soll diese Substanz für die erfindungsgemässen, nachstehend erläuterten Zwecke möglichst nicht von der Membran des Peritoneums resorbiert werden.

Wie bereits vorstehend erläutert, ist die durch Osmose im Peritoneum freigesetzte Wassermenge nicht ohne weiteres festzustellen, so dass sich die Ultrafiltration durch die Einrichtung 112 nicht sonderlich einfach gestaltet. In der Regel wird man daher entweder empirisch vorgehen, also die Pumpe 60 mit einer bestimmten Pumprate betreiben, oder aber den druckempfindlichen Gürtel 98 einsetzen. In der nunmehr erläuterten Ausführungsform wird die im Kreislauf 64 und im Peritoneum befindliche Flüssigkeit in erster Näherung als konstant bezeichnet. In dieser Flüssigkeitsmenge ist innerhalb relativ kurzer Zeit die zur Erkennung des Osmosewassers eingesetzte Substanz gleichmässig verteilt, so dass mit Hilfe des Sensors 116 und/oder 118 eine Anfangskonzentration $c_0$ dieser Substanz festgestellt werden kann. In diesem speziellen Fall sind die Sensoren 116 und 118 über die Leitungen 128 und 130 mit dem Ultrafiltrationssteuergerät 132 in Verbindung. Dieses Ultrafiltrationssteuergerät 132 speichert die ursprüngliche Konzentration $c_0$ der Substanz, die mit fortschreitender Osmose kontinuierlich abnimmt, wenn man voraussetzt, dass die Resorption dieser Substanz durch das Peritoneum in erster Näherung vernachlässigbar ist. Demzufolge wird die Anfangskonzentration $c_0$ im Steuergerät 132 gespeichert und mit dem jeweiligen Ist-Wert verglichen. Anschliessend steuert dieses Gerät 132 die Pumpe 60 über die Leitung 134 so lange, bis der ursprüngliche Konzentrationswert wieder erreicht ist.

Als Detektoren kommen dabei übliche Fluoreszenz-, Absorptions-, oder Transmissions-Detektoren in Frage, die auch auf kleinste Substanzmengen ansprechen können. Im übrigen entspricht die Wirkungsweise des in Fig. 2 gezeigten Peritonealdialysegeräts 110 der Wirkungsweise des Geräts 10 gemäss Fig. 1. Es wird also auch hier ebenfalls intermittierend Dialysierflüssigkeit dem Peritoneum entnommen, wobei entweder das in Fig. 2 gezeigte Pumpsystem mit der Pumpe 82 oder das in Fig. 1 gezeigte Pumpsystem mit der Pumpe 70 zum Einsatz kommen kann. Von Bedeutung ist in beiden Fällen, dass die Konzentration der osmotisch wirksamen Substanz im gesamten System ständig auf einen bestimmten Wert eingestellt wird und somit konstant bleibt. Dies ist von Vorteil gegenüber der CAPD-Behandlung, bei der die osmotisch wirksame Substanz in ihrer Wirkung nachlässt, was sowohl auf die Verdünnung infolge Osmose als auch auf die Resorption der osmotisch wirksamen Substanz durch die Membran des Peritoneums zurückzuführen ist. Beides wird mit dem erfindungsgemässen Peritonealdialysegerät 110 verhindert, da einerseits das durch die Osmose entstandene Wasser mittels Ultrafiltration durch den Dialysator 48 abgepumpt werden kann und andererseits die Konzentration der osmotisch wirksamen Substanz stetig nachgesteuert wird. Allerdings ist dabei zu beachten, dass der Patient nicht in einen hyperglycämischen Schock fällt, was u.U. bei der Gabe von zu viel Glucose der Fall sein könnte.

In einer speziellen Variante wird die Osmose daher nur zu Beginn der Behandlung betrieben, d.h. nach einer bestimmten Zeit, innerhalb der die gewünschte Flüssigkeitsmenge durch die Osmose freigesetzt wird, wird die Glucose aus der gesammten Dialysierflüssigkeit dadurch entfernt, dass die Zuführung von Glucosekonzentrat eingestellt wird. Dies hat zur Folge, dass die Glucose durch Diffusion innerhalb kurzer Zeit aus dem gesamten Peritonealkreislauf verschwindet. Insofern kann auch dann die Ultrafiltrationssteuerung eingestellt werden. Es erfolgt danach nur noch die Entfernung von Stoffwechselprodukten durch Diffusion, nicht jedoch mehr die Freisetzung von Wasser mittels Osmose.

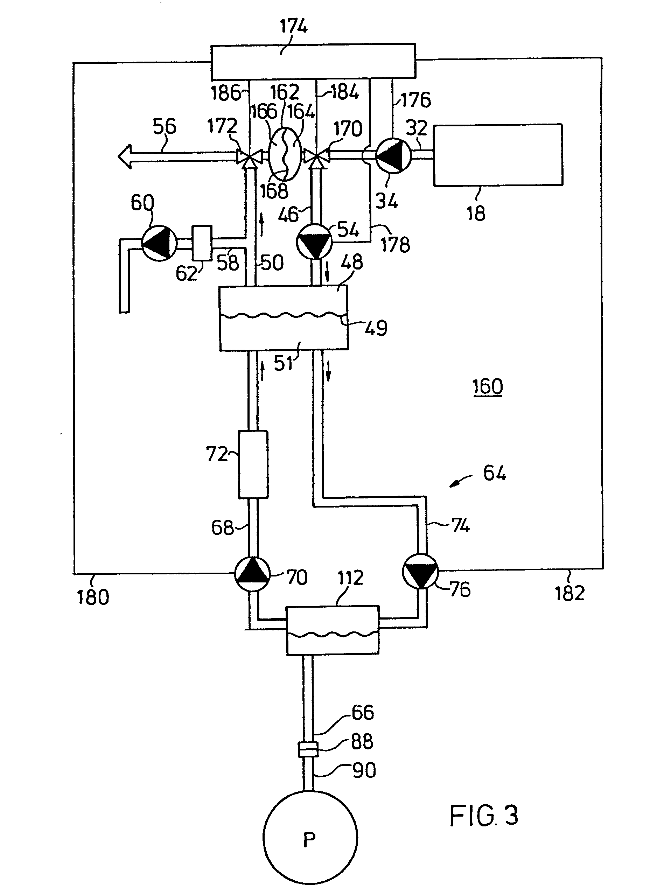

In Fig. 3 ist eine weitere Ausführungsform eines Peritonealdialysegeräts 160 gezeigt, das in seinen wesentlichen Merkmalen von dem Peritonealdialysegerät 140 gemäss Fig. 1 Gebrauch macht, so dass wiederum gleiche Bezugszeichen für gleiche Teile verwendet werden.

Das Peritonealdialysegerät 160 gemäss Fig. 3 weist im Gegensatz zu den in Fig. 1 gezeigten Peritonealdialysegerätennur eine Bilanzkammer 162 auf, die in zwei Bilanzkammerhälften 164 und 166 durch die Membran 168 geteilt ist. Von der Bilanzkammerhälfte 164 geht wiederum die Leitung 32 ab, die mit der Einheit 18 zur Erzeugung der Dialysierflüssigkeit verbunden ist und in die ein 3-Wege-Ventil 170 eingeschaltet ist. Von diesem 3-Wege-Ventil geht die Leitung 46 zum Dialysator 48 ab.

Die andere Bilanzkammerhälfte 166 ist wiederum mit der Leitung 56 verbunden, in die ein zweites 3-Wege-Ventil 172 eingeschaltet ist, dessen zweiter Weg mit der Leitung 50 verbunden ist.

Da das Peritonealdialysegerät 160 nur intermittierend betrieben wird, wird gemäss dieser bevorzugten Ausführungsform nur eine Bilanzkammer 162 benötigt, die im ersten Takt gefüllt bzw. entleert wird und im zweiten Takt in einem geschlossenen Kreislauf an den Hämodialysator angeschlossen wird, damit durch diesen die Dialysierflüssigkeit gepumpt wird.

Um diesen Ablauf zu steuern, ist ein Steuergerät 174 vorgesehen, das mit den Pumpen 34, 54, 70 und 76 über die Leitungen 176, 178, 180 bzw. 182 und mit den 3-Wege-Ventilen 170 und 172 über die Leitungen 184 und 186 verbunden ist.

Das Taktsteuersystem 174 steuert im ersten Takt das Peritonealdialysegerät 160 auf folgende Weise:

Die Pumpe 70 wird in Betrieb genommen und füllt das Speichergefäss 72, während die Pumpe 76 steht. Weiterhin werden die 3-Wege-Ventile 170 und 172 derart geschaltet, dass die Bilanzkammerhälften 164 und 166 unmittelbar mit den Leitungen 32 und 56 verbunden sind. Zugleich wird die Pumpe 34 sowie die Einheit 18 in Betrieb genommen, während die Pumpe 54 stillgesetzt wird. Es beginnt also der Füllvorgang der Bilanzkammer 162, wobei die Membran 168 gemäss der in Fig. 3 gezeigten Ausführungsform nach links wandert und hierdurch die gebrauchte Dialysierflüssigkeit in den Abfluss durch die Leitung 56 verdrängt.

Wenn die Bilanzkammer 162 mit frischer Dialysierflüssigkeit und das Speichergefäss 72 mit verbrauchter Dialysierflüssigkeit gefüllt sind, schaltet das Taktsteuergerät 174 in den zweiten Takt um, wobei die Pumpen 34 und 70 abgeschaltet, die Pumpen 54 und 76 eingeschaltet und die 3-Wege-Ventile 170 und 172 derart umgesteuert werden, dass die Bilanzkammer 162 mit dem Dialysator 48 einen geschlossenen Kreislauf bildet. Demzufolge wandert die Membran durch die Wirkung der Pumpe 54 nach rechts, wobei frische Dialysierflüssigkeit durch den Dialysator gepumpt und als verbrauchte Dialysierflüssigkeit in die linke Bilanzkammerhälfte 166 zurückkehrt.

Sobald das Speichergefäss 72 leergepumpt ist und die Bilanzkammer 162 mit verbrauchter Dialysierflüssigkeit gefüllt ist, steuert das Taktgerät 174 in den ersten Takt zurück, wobei der Füllvorgang von neuem beginnt.

Gemäss den in Fig. 1 und 2 gezeigten Ausführungsformen, bei denen das Dialysegerät 12 permanent läuft, wird bei der in Fig. 3 gezeigten Ausführungsform Dialysierflüssigkeit nur im ersteen Takt zugeführt, während im zweiten Takt die unnötige Förderung an Dialysierflüssigkeit unterbleibt. Demzufolge erfolgt eine erhebliche Einsparung an Dialysierflüssigkeit, die sich letztlich positiv auf die Behandlungskosten niederschlägt.

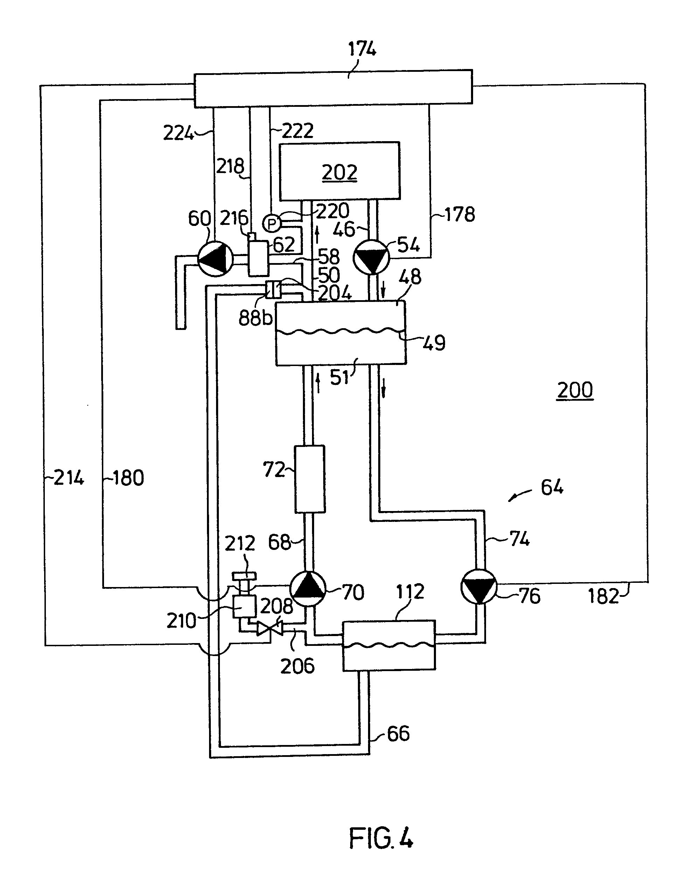

Schliesslich ist in Fig. 4 eine weitere Ausführungsform eines Peritonealdialysegeräts 200 gezeigt, bei dem sowohl ein vollbilanzierendes Dialysegerät 12 gemäss Fig. 1 und 2 als auch eine einzige Bilanzkammer 162 zum Einsatz kommen kann, wie dies in Fig. 3 gezeigt ist. Demzufolge weist das Peritonealdialysegerät 200 eine übliche Einheit zur Bereitstellung von Dialysierflüssigkeit auf, die u.a. auch über ein Desinfektionsprogramm verfügt. Diese Einheit 202 ist über die Leitungen 46 und 50 wiederum mit dem Dialysator 48 verbunden. Die andere Seite des Dialysators weist wiederum den Kreislauf 64 gemäss Fig. 2, 3 oder 4 auf, in den Pumpen 70, 76 bzw. 82 eingeschaltet sind und der einen weiteren Dialysator 112 aufweist. Von diesem Dialysator 112 geht auf der anderen Seite der Membran eine Leitung 66 ab, die entweder, wie in Fig. 1-3 gezeigt, über einen Konnektor 88 mit dem Patienten verbunden ist oder aber, wie in Fig. 4 gezeigt, zur Ablassleitung 50 zurückgeführt ist. Diese Ablassleitung 50 weist ein entsprechenden Konnektorteil 204 auf, das mit einem komplementären Konnektorteil 88b des Konnektors 88 zusammenwirkt.

Somit entspricht dieses Konnektorteil 204 dem Konnektorteil 88a, das sich am Ende des Katheters 90 befindet. Diese beiden Konnektorteile 204 bzw. 88a sind im unbenutzten Zustand jeweils steril dicht verschlossen.

Somit ist aus der in Fig. 4 gezeigten Ausführungsform ersichtlich, dass es sich hier um ein Peritonealdialysegerät 200 handelt, das nicht im Betrieb ist. Ein derartiges Peritonealdialysegerät 200 muss jedoch nach Beendigung der Dialyse gespült, desinfiziert, auf seine Dichtigkeit geprüft, wiederum gespült und anschliessend gefüllt werden, um dann erneut wiederum zum Dialyseeinsatz zu kommen.

Zu diesem Zweck ist das Peritonealdialysegerät 200 in der Leitung 68 mit einer Belüftungsleitung 206 versehen, die vorteilhafterweise unmittelbar stromauf der Pumpe 70 mit der Leitung 68 verbunden ist. In diese Entlüftungsleitung 206 ist ein Ventil 208, eine Ausgleichskammer 210 eingeschaltet und am Ende ein steriles, vorteilhafterweise hydrophobes Belüftungsfilter 212 vorgesehen. Durch dieses Belüftungsfilter lässt sich bei geöffnetem Ventil 208 der Kreislauf 64 sowie die mit diesem Kreislauf 64 verbundenen Kammern der Dialysatoren 48 und 112 belüften.

Zu diesem Zweck sind die Pumpen 70 und 76 wiederum über die Leitungen 180 und 182 mit dem Taktsteuergerät 174 verbunden, das weiterhin über die Leitung 214 mit dem Ventil 208 verbunden ist.

Weiterhin weist das Entgasungsgefäss 62 einen Sensor 216 auf, der eine nichtgezeigte Einrichtung zum Abpumpen überschüssiger Luft aus dem Entgasungsgefäss 62 veranlasst, wie dies in der DE-A-2 838 414 beschrieben ist. Dieser Sensor 216 ist über die Leitung 218 ebenfalls mit dem Taktsteuergerät 174 verbunden.

Weiterhin kann die Leitung 50 mit einem Drucksensor 220 vorteilhafterweise verbunden sein, der über die Leitung 222 mit dem Steuergerät 174 verbunden ist.

Das Peritonealdialysegerät 200 gemäss Fig. 4 wird mit Hilfe des Steuergeräts 174 auf folgende Weise gespült:

Die Einheit 202 stellt in der Spülphase eine Spülflüssigkeit, beispielsweise Wasser, zur Verfügung, wobei jedoch die volumenkonstante Bilanzierung der Einheit 202 nicht aufgehoben wird. Demzufolge bleibt der Kreislauf, der aus der Einheit 202, den Leitungen 46 und 50 und der Dialysatorkammer des Dialysators 48 besteht, konstant, so dass das darin befindliche Flüssigkeitsvolumen imkompressibel bleibt.

Während das Ventil 208 geschlossen bleibt, werden die Pumpen 70 und 76 derart in Betrieb genommen, dass die Förderrate der Pumpe 76 grösser ist als die Förderrate der Pumpe 70. Dies führt dazu, dass in der Dialysatorkammer des Dialysators 48, die mit dem Kreislauf 64 verbunden ist, ein Unterdruck entsteht, so dass Spülflüssigkeit von der einen Kammer durch die Membran in die andere Kammer des Dialysators 48 und von dort in die Leitung 74 gepumpt wird. Da die Pumpe 70 die zugeführte Flüssigkeitsmenge nur teilweise abführen kann, wird der überschüssige Teil durch den zweiten Filter 112 in die Leitung 66 und von dort in die Leitung 50 gepresst, so dass insgesamt ein druckausgleichendes System erhalten wird. Dabei entspricht die durch die Dialysatoren 48 und 112 durchgepresste Flüssigkeitsmenge dem Unterschied der Fördervolumina der Pumpen 70 und 76. Diese Differenz kann in das Steuergerät 174 entsprechend einprogrammiert werden.

An die Spülphase schliesst sich eine Desinfektionsphase an, bei der das Peritonealdialysegerät 200 vollständig mit einer Desinfektionsmittellösung durchgespült wird und anschliessend bis zum erneuten Gebrauch mit diesem Desinfektionsmittel gefüllt bleibt, um eine Verkeimung der Filter und der Leitungen zu verhindern.

Zu diesem Zweck wird die Einheit 202 in den Desinfektionsmittelbetrieb umgeschaltet, so dass anstelle einer Spülflüssigkeit eine Desinfektionsmittellösung zugeführt wird. Dabei läuft die Desinfektionsphase in der gleichen Weise wie die vorstehend beschriebene Spülphase ab, so dass hierauf Bezug genommen wird.

An die Desinfektionsphase schliesst sich unmittelbar vor der erneuten Inbetriebnahme des Peritonealdialysegeräts 200 eine Überprüfung des Kreislaufs 64 auf Dichtigkeit, d.h. auf ein Vorliegen von Lecks an. Zu diesem Zweck ist das Steuergerät 174 mit der Ultrafiltrationspumpe 60 über eine elektrische Leitung 224 verbunden.

In der Überprüfungsphase ist im Gegensatz zur Spül- und Desinfektionsphase die Pumpe 54 und die Einheit 202 nicht in Betrieb. Zur Überprüfung des Kreislaufs 64 wird vom Steuergerät 174 das Ventil 208 geöffnet und die Ultrafiltrationspumpe 60 mit einer vorbestimmten Förderrate in Betrieb genommen. Hierdurch wird die im Kreislauf 64, bestehend aus den Leitungen 68 und 74 und den mit diesen Leitungen verbundenen Dialysatorkammern, enthaltene Flüssigkeit mit Hilfe der Ultrafiltrationspumpe 60 abgepumpt. Zugleich wird durch das Belüftungsfilter 212 Luft in diesen Kreislauf 64 eingepumpt, so dass das Abpumpen in dieser Pumpphase praktisch keinen Unterdruck am Drucksensor 220 entstehen lässt. Sobald der Kreislauf 64 mit Luft gefüllt ist, steigt der Unterdruck langsam an, was auf die Nachgiebigkeit (Compliance) der Membranen der Dialysatoren 48 und 112 zurückzuführen ist. Der von der Ultrafiltrationspumpe 60 erzeugte Unterdruck vermag jedoch nicht die in den Kreislauf 64 eingepumpte Luft durch die mit Wasser benetzten Membranen zu pumpen, so dass am Ende der Nachgiebigkeitsphase ein im wesentlichen konstantes Unterdruckniveau zwischen –200 und 600 mbar erreicht wird. Dieser Verlauf lässt sich mit Hilfe des Drucksensors und dem damit verbundenen Steuergerät 174 bestimmen, das nach einer vorbestimmten Pumpzeit den Kreislauf 64 als dicht und somit steril ansieht, wenn ein vom Atmosphärendruck abweichender Unterdruck erreicht ist. Ein solcher Unterdruck lässt sich nach einer vorbestimmten Pumpphase dann nicht erreichen, wenn ein Riss in der Membran oder eine undichte Stelle im Schlauchsystem vorliegt, was zur Folge hat, dass die gesamten Wegwerfteile ausgetauscht werden müssen.

In einer weiteren Ausführungsform wird anstelle des Drucksensors 220 der Niveausensor 216 des Entlüftungsgefässes 62 benutzt. Bekanntlich schaltet dieser Niveausensor 216 eine nichtgezeigte Entlüftungspumpe ein, wenn durch Zuführung von zuviel Luft das Flüssigkeitsniveau im Entlüftungsgefäss 62 den Sensor 216 betätigt. Sofern also in den Dialysatoren 48 und 112 Membranrisse vorliegen sollten, wird der Niveausensor 216 und damit das Steuergerät 174 betätigt.

Im übrigen kann auch der Druckzeitverlauf mit dem Drucksensor 220 zur Bestimmung der Dichtigkeit des Peritonealdialysegeräts 200 dienen, da dieser Verlauf bei einer bestimmten Pumprate jeweils charakteristisch ist.

Anzumerken ist, dass während dieser Pumpphase beide Pumpen 70 und 76 den Kreislauf 64 freigeben müssen, d.h. in Betrieb genommen werden.

Nach Abschluss der Prüfphase schliesst sich eine Spül/Füllphase an, wobei die Ultrafiltratpumpe 60 abgeschaltet wird, jedoch das Ventil 208 geöffnet bleibt. Es wird jedoch nur zunächst die Pumpe 76 eingeschaltet, während die Pumpe 70 steht. Weiterhin wird aus der Einheit 202 mit Hilfe der Pumpe 54 wiederum Spülflüssigkeit zur Verfügung gestellt. Vorteilhafterweise geht dabei diese Einheit 202 von dem vorstehend geschilderten geschlossenen, also volumenstarren zu einem offenen Kreislauf über, so dass das ganze System ohne Schwierigkeiten gefüllt werden kann.

Die Pumpe 76 wird dabei so lange betrieben, bis am Sterilfilter 212 Spülflüssigkeit auftaucht. Anschliessend wird die Pumpe 70 in Betrieb genommen, um die restliche Luft aus dem Kreislauf zu entfernen. Dabei ist die Förderrate der Pumpe 70 vorteilhafterweise geringer als die Förderrate der Pumpe 76. Dies führt dazu, dass stromab der Pumpe 76 ein Überdruck entsteht, mit der Folge, dass die überschüssige Luft durch das Sterilfilter 212 abgedrückt wird.

Des weiteren wird die in der Leitung 66 enthaltene Desinfektionsmittellösung unter Druck gehalten und wird somit aus dieser Leitung 66 entfernt und durch Spüllösung ersetzt.

Sobald sichergestellt ist, dass das gesamte System mit Spüllösung gefüllt ist, erfolgt die Umschaltung in der Einheit 202 auf die Dialysierflüssigkeit, die bei offener oder geschlossener Einheit 202 zugeführt werden kann.

Sobald das Peritonealdialysegerät 200 mit der Dialysierflüssigkeit gefüllt ist, wird die Leitung 66 von der Leitung 50 durch Öffnen des Konnektors 88b gelöst und mit dem Katheter 90 des Patienten verbunden.

Hierauf erfolgt das Einfüllen einer bestimmten Menge Dialysierflüssigkeit in den Peritonealraum P des Patienten, was wiederum in der offenen Stellung der Einheit 202 erfolgt. Vorteilhafterweise werden durch das Steuergerät 174 nur die Pumpen 54 und 76 in Betrieb genommen, durch die eine vorbestimmte Menge Dialysierflüssigkeit in den Peritonealraum des Patienten eingefüllt wird.

Sobald diese Menge eingefüllt ist, geht die Einheit 202 in den geschlossenen, volumenkonstanten Kreislauf über und das Peritonealdialysegerät 200 wird in der vorstehend beschriebenen Weise bei der Dialyse des Patienten eingesetzt.

Anzumerken ist, dass der Übergang von der geschlossenen auf eine offene Betriebsweise eines bilanzierten Systems in der DE-A-2 838 414 beschrieben ist.

Es ist weiterhin denkbar, dass der Kreislauf 64, bestehend aus dem Dialysator 48, den Leitungen 68 und 74 sowie dem Dialysefilter 112, und die hiervon abgehenden Leitungen in einer Kassette zusammengefasst sind, so dass diese als selbständig handelbares Teil vertrieben werden kann.

Schliesslich kann auch in dem Gefäss 142 für eine Zusatzflüssigkeit die Menge Peritonealspülflüssigkeit vorgelegt werden, die unmittelbar in den Peritonealraum eingepumpt werden soll, so dass sich hierdurch die vorstehend beschriebene Füllphase im offenen System erübrigt.

**Patentansprüche**

1. Peritonealdialysegerät mit wenigstens einem Dialysator (48), der durch eine Membran (49) in eine erste und eine zweite Kammer geteilt ist, wobei die erste Kammer in einen ersten Kreislauf (64) geschaltet ist, an den eine einlumige Katheteranschlusseinrichtung (66, 88, 90) und ein Speicher (72) angeschlossen sind und der eine Einrichtung zum getakteten Fördern der Dialysierflüssigkeit aufweist, und die zweite Kammer über einen zweiten Kreislauf, in den eine Pumpe (54) eingeschaltet ist, mit einer Vorrichtung (12, 202) zur Bereitstellung von Dialysierflüssigkeit verbunden ist, dadurch gekennzeichnet, dass zusätzlich ein Dialysefilter (112) vorgesehen ist, dessen erste Kammer in den ersten Kreislauf (64) stromauf des Speichers (72) geschaltet ist und an dessen zweite Kammer die Katheteranschlusseinrichtung (66, 88, 90) angeschlossen ist.

2. Gerät nach Anspruch 1, dadurch gekennzeichnet, dass der Dialysator (48) ein Hämodialysator ist, dessen Trenngrenze bei 5000 - 10 000 MG liegt.

3. Gerät nach Anspruch 1 oder 2, dadurch gekennzeichnet, dass das Dialysefilter (112) ein Hämofilter mit einer Trenngrenze in einem Bereich von 20 000 - 400 000 MG ist.

4. Gerät nach Anspruch 1, gekennzeichnet durch eine Einrichtung (60) zur Entziehung von Ultrafiltrat, die an der Vorrichtung (12) zur Bereitstellung der Dialysierflüssigkeit vorgesehen ist.

5. Gerät nach Anspruch 1, dadurch gekennzeichnet, dass in der Zuleitung (74) zum Dialysefilter (112) eine erste Pumpe (76) und in der Ableitung (68) vom Dialysefilter (112) eine zweite Pumpe (70) vorgesehen sind und alternierend betrieben werden und jeweils im Ruhezustand diese Leitungen (68, 74) sperren.

6. Gerät nach Anspruch 5, dadurch gekennzeichnet, dass die erste und zweite Pumpe (70, 76) peristaltische Pumpen sind.

7. Gerät nach Anspruch 1 oder 2, dadurch gekennzeichnet, dass die Vorrichtung (12) zur Bereitstellung der Dialysierflüssigkeit als volumenkonstantes, bilanzierendes System ausgebildet ist.

8. Gerät nach einem der Ansprüche 1 bis 7, dadurch gekennzeichnet, dass stromab der ersten Kammer des Dialysators (48) in die zum Dialysefilter (112) führende Leitung (74) eine Tropfkammer (78) eingeschaltet ist, die eine Einrichtung (92) zur Entfernung von Luft und einen Niveausensor (94) aufweist.

9. Gerät nach einem der Ansprüche 1 bis 8, dadurch gekennzeichnet, dass in den ersten Kreislauf (64), in den das Dialysefilter (112) eingeschaltet ist, eine Substanz vorgesehen ist, deren Molekulargewicht oberhalb der Trenngrenze des Dialysators (48) und des Dialysefilters (112) liegt.

10. Gerät nach Anspruch 9, dadurch gekennzeichnet, dass an der vom Dialysefilter (112) zum Katheter (90) führenden Leitung (66) ein erster Sensor (116), an der vom Dialysefilter (112) abgehenden Leitung (68) ein zweiter Sensor (118) und/oder an der im zweiten Kreislauf vorgesehenen, vom Dialysator (48) abgehenden Leitung (50) ein dritter Sensor (120) vorgesehen sind, die mit einem Überwachungsgerät (114) verbunden sind.

11. Gerät nach einem der Ansprüche 1 bis 8, dadurch gekennzeichnet, dass im ersten Kreislauf (64) eine Substanz vorgesehen ist, deren Molekulargewicht grösser als die Trenngrenze des Dialysators (48) jedoch kleiner ist als die Trenngrenze des Dialysefilters (112).

12. Gerät nach einem der Ansprüche 1 bis 11, dadurch gekennzeichnet, dass die zum Dialysefilter (112) führende Leitung (74) über eine erste Leitung (144), in die eine dritte Pumpe (146) oder eine Klemme (148) eingeschaltet ist, mit einem Gefäss (142) für eine Zusatzflüssigkeit verbunden ist, wobei die dritte Pumpe (146) oder die Klemme (148) synchron zur Inbetriebnahme der ersten Pumpe (76) betätigt werden.

13. Gerät nach Anspruch 12, dadurch gekennzeichnet, dass die dritte Pumpe (146) oder die Klemme (148) mit einem Ultrafiltrationssteuergerät (102) verbunden ist, die die Einrichtung (60) zur Entziehung von Ultrafiltrat zur Entnahme einer bestimmten Ultrafiltratmenge veranlasst, die der aus dem Gefäss (142) abgeführten Flüssigkeitsmenge entspricht.

14. Gerät nach einem der Ansprüche 1 bis 4 und 6 bis 13, dadurch gekennzeichnet, dass die Vorrichtung (12) zur Bereitstellung von Dialysierflüssigkeit eine erste Bilanzkammerr (162) aufweist, die getaktet zur Arbeitsweise der ersten und zweiten Pumpe (76, 70) unter Verdrängung der verbrauchten Dialysierflüssigkeit gefüllt (1. Takt) und deren Inhalt mit Hilfe der in den zweiten Kreislauf eingeschalteten Pumpe (54) durch den Dialysator (48) gepumpt wird (2. Takt), wobei im ersten bzw. zweiten Takt die zweite Pumpe (70) bzw. die erste Pumpe (76) läuft.

15. Gerät nach einem der Ansprüche 1 bis 14, dadurch gekennzeichnet, dass die vom Dialysefilter (112) zum Katheter (90) führende Leitung (66) mit der im zweiten Kreislauf vom Dialysator (48) abgehenden Ablassleitung (50) lösbar in der Ruhestellung verbunden ist, die erste und zweite Pumpe (76, 70) mit einem Steuergerät (174) verbunden sind, das in der Spül- und Desinfektionsphase die erste und zweite Pumpe (76, 70) derart steuert, dass die Pumprate der ersten Pumpe (76) gegenüber der Pumprate der zweiten Pumpe (70) grösser ist.

16. Gerät nach einem der Ansprüche 1 bis 15, dadurch gekennzeichnet, dass zwischen der zweiten Pumpe (70) und dem Dialysefilter (112) von der vom Dialysefilter (112) abgehenden Leitung (68) eine zweite Leitung (206) abgeht, in die ein mit dem Steuergerät (174) verbundenes Ventil (208) eingeschaltet ist und deren Ende mit einem hydrophoben Filter (212) verschlossen ist, und die Einrichtung (60) zur Entziehung von Ultrafiltrat mit dem Steuergerät (174) verbunden ist.

17. Gerät nach Anspruch 15 oder 16, dadurch gekennzeichnet, dass das Steuergerät (174) in der Prüfphase das Ventil (208) öffnet und die Einrichtung (60) zur Entziehung von Ultrafiltrat in Betrieb nimmt und mit einem Drucksensor (220), der mit der vom Dialysator (48) im zweiten Kreislauf abgehenden Leitung (50) verbunden ist, und/oder mit einem Niveausensor (216), der an einem im zweiten Kreislauf vorgesehenen Entgasungsgerät (62) angeordnet ist, verbunden ist und hierdurch das Druck/Zeit-Verhältnis oder ein Luftleck bestimmt.

18. Gerät nach einem der Ansprüche 1 bis 17, dadurch gekennzeichnet, dass die Vorrichtung (12, 202) zur Bereitstellung der Dialysierflüssigkeit in der Füllphase in die offene Betriebsweise schaltbar ist, das Ventil (208) zur Entfernung der Luft geöffnet ist, die erste Pumpe (76) so lange eingeschaltet ist, bis am hydrophoben Filter (212) Flüssigkeit auftaucht und die zweite Pumpe (70) anschliessend in Betrieb genommen wird, wobei die Förderrate der zweiten Pumpe (70) geringer ist als die Förderrate der ersten Pumpe (76).

19. Gerät nach einem der Ansprüche 1 bis 18, dadurch gekennzeichnet, dass die Steuervorrichtung (174) die Vorrichtung (202) zur Bereitstellung der Dialysierflüssigkeit nach dem Anschluss der vom Dialysefilter (112) zum Katheter (90) führenden Leitung (66) an den Katheter (90) des Patienten derart steuert, dass eine bestimmte Flüssigkeitsmenge durch diese Leitung (66) und den Katheter (90) abgegeben wird.

20. Gerät nach einem der Ansprüche 10 bis 19, dadurch gekennzeichnet, dass der erste Sensor (116) und/oder der zweite Sensor (118) mit einem Ultrafiltrationssteuergerät (132) verbunden sind, mit dem durch Vergleich mit der Anfangskonzentration der Substanz zur Steuerung der Ultrafiltration die Einrichtung (60) zur Entziehung von Ultrafiltrat steuerbar ist.

**Claims**

1. A peritoneal dialysis apparatus comprising at least one dialyzer (48), which is divided by a membrane (49) into a first and second chamber, whereby the first chamber is arranged in a first circuit (64) to which a single lumen catheter (66, 88, 90) and a storage (72) are joined and which comprises a means for timed conveying of the dialysis liquid, and the second chamber is joined via a second circuit, into which a pump (54) is arranged, to a unit (12, 202) for supplying dialysis liquid, characterized in that additionally a dialysis filter (112) is provided the first chamber of which is arranged in the first circuit (64) streamup the storage (72) and to the second chamber of which the catheter (66, 88, 90) is connected.

2. Apparatus according to claim 1, characterized in that the dialyzer (48) is a hemodialyzer the cut-off limit of which is 5000 to 10 000 MW.

3. Apparatus according to claim 1 or 2, characterized in that the dialysis filter (112) is a hemofilter with a cut-off limit within a range of 20 000 - 400 000 MW.

4. Apparatus according to claim 1, characterized by a means (60) for withdrawing ultrafiltrate which is provided at the unit (12) for supplying the dialysis liquid.

5. Apparatus according to claim 1, characterized in that in the duct (74) to the dialysis filter (112) a first pump (76) and in the duct (68) from the dialysis filter (112) a second pump (70) are provided and are operated alternatingly and shutting off said ducts (68, 74), respectively in state of rest.

6. Apparatus according to claim 5, characterized in that said first and second pump (70, 76) are peristaltic pumps.

7. Apparatus according to claim 1 or 2, characterized in that said unit (12) for supplying the dialysis liquid is embodied as constant-volume, balancing system.

8. Apparatus according to any of claims 1 to 7, characterized in that streamup the first chamber of the dialyzer (48) a drip chamber (78) is arranged in the duct (74) leading to the dialysis filter (112), said drip chamber comprising a means for venting air and a level sensor (94).

9. Apparatus according to any of claims 1 to 8, characterized in that in the first circuit (64) in which the dialysis filter (112) is arranged, a substance is provided whose molecular weight is above the cut-off limit of the dialyzer (48) and the dialysis filter (112).

10. Apparatus according to claim 9, characterized in that at the duct (66) leading from the dialysis filter (112) to the catheter (90) a first sensor (116), at the duct (68) leading off the dialysis filter (112) a second sensor (118) and/or at the duct (50) provided in the second circuit and leading off the dialyzer (48) a third sensor (120) are provided which are connected with a monitoring device (114).

11. Apparatus according to any of claims 1 to 8, characterized in that in the first circuit (64) a substance is provided whose molecular weight is greater than the cut-off limit of the dialyzer (48), however, smaller than the cut-off limit of the dialysis filter (112).

12. Apparatus according to any of claims 1 to 11, characterized in that the duct (74) leading to the dialysis filter (112) is joined via a first duct (144), in which a third pump (146) or a clamp (148) is arranged, to a vessel (142) for an additional liquid, whereby the third pump (146) or the clamp (148) are operated synchronously with the starting of the first pump (76).

13. Apparatus according to claim 12, characterized in that the third pump (146) or the clamp (148) is joined with an ultrafiltration controller (102) which causes the unit (60) for withdrawal of ultrafiltrate to discharge a defined amount of ultrafiltrate which corresponds to the amount of liquid discharged from the vessel (142).

14. Apparatus according to any of claims 1 to 4 and 6 to 13, characterized in that the unit (12) for supplying dialysis liquid comprises a first balancing chamber (162) which in a cycle in step with the operation of the first and second pump (76, 70), by removing the used-up dialysis liquid is filled (first stroke) and the content of which is pumped by means of the pump (54) arranged in the second circuit through the dialyzer (48) (second stroke), whereby in the first stroke the second pump (70) and in the second stroke the first pump (76) is being operated.

15. Apparatus according to any of claims 1 to 14, characterized in that the duct (66) leading off said dialysis filter (112) to said catheter (90) is detachably joined in the resting position of the exit duct (50) passing in the second circuit off the dialyzer (48), that the first and second pump (76, 70) are joined with a controller (174) which in the swilling and disinfection phase controls the first and second pump such that the pumping rate of the first pump (76) is greater in comparison with the pumping rate of the second pump (70).

16. Apparatus according to any of claims 1 to 15, characterized in that between the second pump (70) and the dialysis filter (112) a second duct (206) passes off the duct (68) passing off the dialysis filter (112) in which second duct a valve (208) is arranged and joined with said controller (174) and whose end is closed by a hydrophobic filter (212), and the unit (60) for withdrawal of ultrafiltrate is joined with the controller (174).

17. Apparatus according to claim 15 or 16, characterized in that the controller (174) opens the valve (208) during the control phase and starts the operation of the unit (60) for withdrawal of ultrafiltrate and is joined with a pressure sensor (220) being joined with the duct (50) passing off the dialyzer (48) in the second circuit, and/or with a level sensor (216) being arranged at a degassing vessel (62) and which hereby determines the pressure/time-relation or an air leak.

18. Apparatus according to any of claims 1 to 17, characterized in that the unit (12, 202) for supplying the dialysis liquid may be switched in the filling phase into the open operation mode, the valve (208) for withdrawal of air is opened until at the hydrophobic filter (212) liquid appears and the second pump (70) is subsequently put into operation, whereby the pumping rate of the second pump (70) is smaller than the pumping rate of the first pump (76).

19. Apparatus according to any of claims 1 to 18, characterized in that the controller (174) controls the unit (202) for supplying the dialysis liquid after the connection of the duct (66) leading from the dialysis filter (112) to the catheter (90) to the catheter (90) of the patient such that a defined amount of liquid is discharged through said duct (66) and the catheter (90).

20. Apparatus according to any of claims 10 to 19, characterized in that the first sensor (116) and/or the second sensor (118) are connected with an ultrafiltration controller (132) by which through comparison with the initial concentration of the substance for controlling the ultrafiltration the unit (60) for withdrawal of ultrafiltrate is controllable.

**Revendications**

1. Appareil de dialyse péritonéale comprenant au moins un dialyseur (48) qu'une membrane (49) divise en une première et une seconde chambre, la première chambre étant insérée dans un premier circuit (64) auquel sont reliés un dispositif (66, 88, 90) de raccordement d'un cathéter à un canal et un réservoir (72) et qui comprend un dispositif pour le débit cadencé du liquide dialysant, et la seconde chambre étant reliée par un circuit dans lequel est insérée une pompe (54) à un dispositif (12, 202) fournissant le liquide dialysant, ledit appareil étant caractérisé en ce qu'un filtre de dialyse (112) est en outre prévu, dont la première chambre est insérée dans le premier circuit (64) en aval du réservoir (72) et dont la seconde chambre est reliée au dispositif (66, 88, 90) de raccordement du cathéter.

2. Appareil selon revendication 1, caractérisé en ce que le dialyseur (48) est un hémodialyseur dont la limite de séparation est de PM 5000 - 10 000.

3. Appareil selon une des revendications 1 ou 2, caractérisé en ce que le filtre de dialyse (112) est un hémofiltre dont la limite de séparation est située sur une plage de PM 20 000 - 400 000.

4. Appareil selon revendication 1, caractérisé par un dispositif (60) d'extraction de l'ultrafiltrat, prévu sur le dispositif (12) fournissant le liquide dialysant.

5. Appareil selon revendication 1, caractérisé en ce qu'une première pompe (76) est prévue dans la canalisation (74) d'alimentation du filtre de dialyse (112) et une deuxième pompe (70) dans la canalisation (68) de départ du filtre de dialyse (112), lesdites pompes fonctionnant alternativement et bloquant au repos chacune de ces canalisations (68, 74).

6. Appareil selon revendication 5, caractérisé en ce que la première et la deuxième pompe (70, 76) sont des pompes péristaltiques.

7. Appareil selon une des revendications 1 ou 2, caractérisé en ce que le dispositif (12) fournissant le liquide dialysant est réalisé sous forme d'un système à bilan et volume constant.

8. Appareil selon une quelconque des revendications 1 à 7, caractérisé en ce qu'une chambre à gouttes (78) est insérée dans la canalisation (74) aboutissant au filtre de dialyse (112), en aval de la première chambre du dialyseur (48), et comprend un dispositif (92) d'extraction de l'air et un capteur de niveau (94).

9. Appareil selon une quelconque des revendications 1 à 8, caractérisé en ce qu'une substance, dont le poids moléculaire est supérieur à la limite de séparation du dialyseur (48) et du filtre de dialyse (112), est prévue dans le premier circuit (64) contenant le filtre de dialyse (112).

10. Appareil selon revendication 9, caractérisé en ce qu'un premier capteur (116) est prévu sur la canalisation (66) reliant le filtre de dialyse (112) au cathéter (90), un deuxième capteur (118) est prévu sur la canalisation (68) partant du filtre de dialyse (112) et/ou un troisième capteur (120) est prévu sur la canalisation (50) partant du dialyseur (48) dans le second circuit, les trois capteurs étant reliés à un appareil de contrôle (114)..

11. Appareil selon une quelconque des revendications 1 à 8, caractérisé en ce qu'une substance, dont le poids moléculaire est supérieur à la limite de séparation du dialyseur (48), mais inférieur à la limite de séparation du filtre de dialyse (112), est prévue dans le premier circuit (64).

12. Appareil selon une quelconque des revendications 1 à 11, caractérisé en ce que la canalisation (74) aboutissant au filtre de dialyse (112) est reliée par une première canalisation (144) dans laquelle est insérée une troisième pompe (14) ou une pince (148), à un réservoir (142) de liquide additionnel, la troisième pompe (146) ou la pince (148) étant actionnée au synchronisme avec la mise en service de la première pompe (76).

13. Appareil selon revendication 12, caractérisé en ce que la troisième pompe (146) ou la pince (148) est reliée à un appareil de commande d'ultrafiltration (102), qui conduit le dispositif (60) à extraire une quantité donnée d'ultrafiltrat, égale à la quantité de liquide évacuée du réservoir (142).

14. Appareil selon une quelconque des revendications 1 à 4 et 6 à 13, caractérisé en ce que le dispositif (12) fournissant le liquide dialysant comprend une première chambre de bilan (162), qui est remplie en cadence avec le fonctionnement de la première et de la deuxième pompe (76, 70), avec refoulement du liquide dialysant épuisé (temps 1) et dont le contenu est pompé à travers le dialyseur (48) par la pompe (54) insérée dans le second circuit (temps 2), la deuxième pompe (70) ou la première (76) fonctionnant pendant le premier ou le second temps.

15. Appareil selon une quelconque des revendications 1 à 14, caractérisé en ce que la canalisation (66) reliant le filtre de dialyse (112) au cathéter (90) est reliée de façon détachable et en position de repos à la canalisation d'évacuation (50) partant du dialyseur (48) dans le second circuit; et la première et la deuxième pompe (76, 70) sont reliées à un appareil de commande (174) qui, dans les phases de rinçage et de désinfection, commande la première et la deuxième pompe (76, 70) de façon que le débit de la première (76) soit supérieur à celui de la deuxième (70).

16. Appareil selon une quelconque des revendications 1 à 15, caractérisé en ce qu'entre la deuxième pompe (70) et le filtre de dialyse (112), une deuxième canalisation (206) part de la canalisation (68) partant du filtre de dialyse (112), contient une vanne (208) reliée à l'appareil de commande (174) et est obturée à l'extrémité par un filtre hydrophobe (212); et le dispositif (60) d'extraction de l'ultrafiltrat est relié à l'appareil de commande (174).

17. Appareil selon une des revendications 15 ou 16, caractérisé en ce que l'appareil de commande (174), dans la phase de contrôle, ouvre la vanne (208) et met en service le dispositif (60) d'extraction de l'ultrafiltrat, est relié à un capteur de pression (220) relié à la canalisation (50) partant du dialyseur (48) dans le second circuit et/ou est relié à un capteur de niveau (216) disposé sur l'appareil de dégazage (62) prévu dans le second circuit et détermine ainsi la variation pression/temps ou une fuite d'air.

18. Appareil selon une quelconque des revendications 1 à 17, caractérisé en ce que le dispositif (12, 202) fournissant le liquide dialysant est commutable en mode ouvert dans la phase de remplissage; la vanne (208) d'évacuation de l'air est ouverte; la première pompe (76) demeure branchée jusqu'à l'apparition de liquide sur le filtre hydrophobe (212); et la deuxième pompe (70) est mise ensuite en service, son débit étant inférieur à celui de la première pompe (76).

19. Appareil selon une quelconque des revendications 1 à 18, caractérisé en ce qu'après le raccordement au cathéter (90) du patient de la canalisation (66) reliant le filtre de dialyse (112) au cathéter (90), le dispositif (174) commande le dispositif (202) fournissant le liquide dialysant de façon qu'une quantité donnée de liquide soit délivrée par cette canalisation (66) et le cathéter (90).

20. Appareil selon une quelconque des revendications 10 à 19, caractérisé en ce que le premier capteur (116) et/ou le deuxième (118) sont reliés à un appareil de commande d'ultrafiltration (132) qui, par comparaison avec la concentraton initiale de la substance de commande de l'ultrafiltration, commande le dispositif (60) d'extraction de l'ultrafiltrat.

FIG.1

FIG. 2

FIG. 3

FIG.4